Europäisches Patentamt

European Patent Office

Office européen des brevets

(19)

(11) **EP 1 188 825 A1**

(12) **EUROPEAN PATENT APPLICATION**

(43) Date of publication:
 **20.03.2002 Bulletin 2002/12**

(51) Int Cl.⁷: **C12N 5/10**, A61K 35/14,
 A61P 35/00, A61P 31/00

(21) Application number: **00203232.4**

(22) Date of filing: **18.09.2000**

(84) Designated Contracting States:
 **AT BE CH CY DE DK ES FI FR GB GR IE IT LI LU
 MC NL PT SE**
 Designated Extension States:
 **AL LT LV MK RO SI**

(71) Applicant: **Universiteit Leiden
 2312 AV Leiden (NL)**

(72) Inventors:
 • **Falkenburg, Johan Herman Frederik
  2341 ET Oegstgeest (NL)**
 • **Heemskerk, Maria Huberta Margaretha
  2172 JW Sassenheim (NL)**

(74) Representative: **Prins, Adrianus Willem et al
 Vereenigde,
 Nieuwe Parklaan 97
 2587 BN Den Haag (NL)**

(54) **T cell receptor transfer into a candidate effector cell or a precursor thereof**

(57) The present invention provides means and methods for generating effector cells that can be used for, for instance, adoptive immuno therapy. In one aspect is provided a method for providing a candidate effector cell with an antigen specific effector activity comprising providing said cell or a precursor thereof with a recombinant αβ T cell receptor specific for said antigen or a functional part, derivative and/or analogue of said receptor. In a preferred embodiment said effector cell comprises cytotoxic activity.

EP 1 188 825 A1

**Description**

The invention relates to the field of medicine. In particular, the invention relates to the field of immuno-therapy.

Many strategies have been tried to utilize the immune system to prevent and/or treat diseases in humans. One of these strategies involves the use of T cells that can mediate targeted destruction specific cells in a host. This experimental therapy is also referred to as adoptive immuno therapy. The present invention provides means and methods that can be used in immuno therapy. Means and methods of the invention are useful for influencing an effector response against practically any type of cell in a body. The invention is exemplified on the basis of human malignancies and more in particular human hemopoietic malignancies. However, this is by no means intended to limit the scope of the invention.

Human malignancies of various origin are classically treated using surgery, radiotherapy and chemotherapy. If no complete eradication of the tumor can be obtained by surgery or radiotherapy, chemotherapy is usually applied to obtain cure or to minimize the tumor load leading to prolonged survival. Although chemotherapy can successfully be applied in many patients with malignancies, the malignancy frequently reoccurs. Continuous treatment with chemotherapy is usually hampered by toxic side effects of these drugs, and the development of resistance of the tumor for these agents. Therefore, alternative strategies need to be developed to obtain cure in the majority of patients with malignancies, in particular in patients with advanced stages of disease.

Several hematological malignancies and solid tumors have been shown to be immunogenic tumors i.e. able to elicit an anti-tumor effector response in patients. Although in some cases the occurrence of such an effector response may coincide with remission of the disease, in most patients these effector responses fail to eradicate the tumor. Apparently, although T cell mediated effector responses against the tumor occur in vivo, the effector reaction is crippled by many factors which may include suppression of an effector response by the tumor cells, the occurrence of tolerance against the malignancy or inability of the T cell compartment to proliferate and expand to sufficient numbers to eradicate the malignancy. Vaccination protocols have been initiated to augment the effector response in patients, but with very limited success. An alternative approach may be to isolate and expand tumor specific T cells in vitro to sufficient numbers and infuse those T cells into the patient to attack and eliminate the tumor. This approach has been successfully explored in the treatment of hematological malignancies.

Allogeneic stem cell transplantation (SCT) has been successfully applied in many patients with hematological malignancies. These allogeneic stem cell grafts do not only contain stem cells, but also donor derived T cells. T cells present in the graft may induce Graft versus Host Disease (GvHD) which is a harmful reaction of these lymphocytes against normal tissues of the recipient. T cell depletion of the donor graft resulted in a decreased frequency of GvHD, but was associated with an increased risk of relapse of the malignancy after transplantation indicating a "graft versus leukemia (GVL)" effect mediated by T cells. It was demonstrated that also in the absence of GvHD, allogeneic T cells in the graft may exhibit a GVL effect. The clinical observations that donor-derived T cells may exhibit a GVL effect after allogeneic SCT have led to the strategy to explore the possibility to use donor derived T lymphocytes directly in the control of hematological malignancies. In patients who relapsed after allogeneic SCT, treatment with donor lymphocyte infusions (DLI) led to complete sustained remissions in the majority of patients with relapsed chronic myeloid leukemia (CML), but also in patients with other hematological malignancies including acute myeloid leukemia (AML), acute lymphoblastic leukemia (ALL), multiple myeloma (MM) and non-Hodgkin's lymphoma (NHL). However, this treatment was frequently accompanied by GvHD. Treatment of patients with T cells with more defined specificity may improve the efficacy with fewer side effects.

Both CD4+ and CD8+ T cells with relative specificity for the malignant cells or specific for minor Histocompatibility antigens (mHag) expressed on hematopoietic cells can be isolated from normal donors and from patients with hematological malignancies after transplantation. In vitro, these cells were capable of lysing the malignant cells from the patient without affecting non-hematopoietic cells from the patient or normal hematopoietic cells from the stem cell donor illustrating relative specificity of these T cells for the malignancy. We have demonstrated that these leukemia reactive or mHag specific CTL can be isolated and expanded in vitro under good manufacturing practice (GMP) conditions. We have demonstrated that these leukemia reactive CTL can be infused into patients with relapsed malignancy after allogeneic SCT without significant side effects, and that these CTL are capable of inducing complete eradication of the tumor. We illustrated that similar leukemia reactive or mHag specific T cells capable of attacking the leukemic (precursor) cells were responsible for successful treatment of patients with DLI after transplantation.

To improve the reproducibility of the in vitro selection and expansion of T cells to be used for eradicating malignant cells in vivo, the fine specificity of the T cell responses has to be defined. In the context of allogeneic SCT, T cells recognizing mHag specifically expressed in hematopoietic cells may result in eradication of the malignancy without affecting donor cells. MHag are peptides derived from polymorphic proteins that can be presented by HLA-molecules on the tumor cells. MHag such as HA-1 and HA-2 that are exclusively expressed on hematopoietic cells including leukemic cells and their progenitors may be appropriate targets for donor derived T cells to treat malignancies in the context of allogeneic SCT. Furthermore, malignancy-specific target peptides like fusionpeptide encoded by neogenes resulting from chromosomal translocations may serve as antigens. In addition, several non-polymorphic antigens including peptides derived from proteinase-3 or WT-1 are being explored as target structures for immunotherapy of hematological malignancies. Tumor associated antigens have been characterized in a variety of solid tumors as well, including breast cancer, melanoma, renal cell carcinoma, prostate cancer, cervical cancer and others. Enrichment of T cells recognizing specific antigens is usually performed by in vitro stimulation of the T cells with specific tumor cells or with antigen presenting cells (APC) loaded with tumor associated proteins or specific peptides. Additional selection of specific T cells can be performed using tetrameric complexes composed of the specific peptide in combination with the HLA-molecules that are the restriction element for specific T cell recognition. Alternatively, antigen specific T cells can be isolated based on their ability to specifically secrete certain cytokines. These methods lead to enrichment of specific T cells, although the purity of the antigen specific T cells is frequently limited. Clonal selection may result in complete defined T cell specificities, but is logistically hardly feasible for broad applications.

Several other factors may limit the possibility to efficiently generate defined antigen specific T cell populations in each patient or donor. In patients, the effector response may be skewed or suppressed resulting in the inability to expand the required T cell populations. Furthermore, the T cell repertoire is not always optimal for the generation of the desired T cells with sufficient affinity and avidity of the TCR. In addition, the composition and the fine specificity of the T cell populations will be hard to control. Even when composition and specificity can be defined, it may not be possible to expand the specific T cell lines to sufficient amounts to use these T cells for adoptive immunotherapy in all patients. Specificity, expandability and reproducibility are essential for broad application of adoptive immunotherapy in the treatment of cancer and other diseases.

It is clear, also from the above, that many approaches have been tried to arrive at effector cells that are capable of performing an effector function in a predictable way. However, none have led to a satisfactory solution. The present invention now provides a method for generating an effector cell comprising providing a candidate effector cell or a precursor thereof, with a recombinant $\alpha\beta$ T cell receptor or a functional part, derivative and/or analogue of said receptor. An effector cell can display antigen specific activity. Antigen specificity effector cells is mediated by said $\alpha\beta$ T cell receptor which is capable of binding antigen presented in the context of an HLA molecule. An effector cell can initiate, stimulate, inhibit and/or prevent an immune response in a host. An effector cell can also comprise antigen specific cytotoxic activity. A candidate effector cell is a cell not comprising effector activity or not comprising a recognized effector activity or give rise to specific cytokine production. In a preferred embodiment said candidate effector cell is a primary cell, preferably a primary T cell. A primary cell is a cell isolated from an individual. Said primary cell may be cultured outside the body of said individual as long as the properties of said cell are not irreversibly altered such that said cell becomes immortalized. A candidate cell can be obtained from any part of the body of an individual. Preferably, said candidate cell is obtainable from bone marrow, a lymphoid organ or blood.

In a preferred embodiment of the invention said effector cell does not express a functional endogenous $\alpha\beta$ T cell receptor. An $\alpha$ chain of a T cell receptor is typically capable of pairing with any $\beta$ chain and vise versa. Expression of more than one $\alpha$ chain or $\beta$ chain in a cell can thus lead to the formation of several different $\alpha\beta$ T cell receptors. By way of example, the expression of two $\alpha$ and two $\beta$ chain in a cell could lead to the formation of 4 different $\alpha\beta$ T cell receptors. Each of these 4 receptors can comprise a different antigen specificity. Thus if one would like to obtain expression of an additonal $\alpha\beta$ T cell receptor, the effector cell typically would express 2 $\alpha$ and 2 $\beta$ chains. This can result in the formation of 4 different $\alpha\beta$ T cell receptors, two of which would have the desired specificity and two of which would not have the desired specificity. Very often it will not be known what specificity these other two $\alpha\beta$ T cell receptors will have. It could be a specificity that is detrimental.

It is possible to determine the antigen specificity of a T cell receptor. It is therefore also possible to test for detrimental T cell receptors upon expression of more than one $\alpha$ and/or $\beta$ chain in a cell. If a detrimental T cell receptor specificity is observed, one can choose for instance, not to use the cell comprising said detrimental receptor. It is of course also possible to test in advance whether the combined expression of two or more $\alpha\beta$ T cell receptor in a cell would lead to the formation of a detrimental T cell receptor. Combinations leading to the formation of a detrimental $\alpha\beta$ T cell receptor can thereby be avoided if necessary.

However, typically the testing of antigen specificity is time consuming. For transplantation purposes, especially in immuno therapy, time is not available. A patient often needs treatment as soon as possible in order to at least avoid unnecessary suffering or progression of disease. Some effector cells, particularly cytotoxic T cells, naturally express $\alpha\beta$ T cell receptors, prior to being provided with a T cell receptor of the invention. Typically such endogenously expressed T cell receptors comprise an unknown specificity. Moreover, combinations of endogenously expressed $\alpha$ or $\beta$ chains with an $\alpha$ or $\beta$ chain of a recombinant T cell receptor could provide another source of detrimental T cell receptors. To at least in part eliminate this potential source of detrimental T cell receptors it is preferred that said effector cell does not express a functional endogenous T cell receptor $\alpha$ and/or $\beta$ chain.

In the present invention we demonstrate a possibility to circumvent this problem. In one aspect of the invention said candidate effector is a $\gamma\delta$-T cell or a precursor thereof. A $\gamma\delta$-T cell can be provided with effector cell properties by providing said cell with an $\alpha\beta$ T cell receptor. Preferably, said $\gamma\delta$-T cell is derived from peripheral blood. The present invention demonstrates that a γδ-T cell possesses an intracellular machinery to express CD3 which is necessary to transport the TCRαβ complex to the cell surface. At least some γδ-T cells also co-express CD4 or CD8. The present invention demonstrates that it is possible to generate effector cells from γδ-T cell. It is thus demonstrated that it is possible to generate effector cells with a predicted antigen specificity that have no undesired expression of newly formed TCRα and β complexes by pairing with endogenous α and β TCR chains. Since a γδ cell is capable of killing a cell, a new TCRαβ killer T cell is generated following gene transfer of antigen specific TCRα and β chains into this cell. The transfer of a combination of a specific TCRα and β chain into a γδ-T cell leads to the generation of an antigen specific killer cell with the specificity of the transferred TCRαβ complex. We also demonstrate that these newly generated T cells can be expanded to large numbers. Thus by providing a γδ-T cell with an αβ T cell receptor or a functional part, derivative and/or analogue thereof, said γδ-T cell was at least in part, functionally converted into an αβ T cell receptor containing cell. This embodiment of the present invention opens the possibility to redirect any specific TCR into a γδ-T cell or a precursor thereof, and by doing so generating large numbers of antigen specific αβ-T cells. Such cells can be used not only for cellular adoptive immunotherapy for the treatment of malignancies, but also for instance of viral diseases like CMV and EBV. Thus generated effector cells can typically provide any function of a classical αβ T cell. Classical αβ T cells are involved in the induction and stimulation of immune responses, including the induction or stimulation of cytotoxicity against non-self antigens, suppresion of tumors, suppression of immune responses and preventing auto-immune diseases, tolerance against organs transplanted, and many other regulatory functions. In a preferred embodiment of the invention an effector cell generated with a method of the invention comprises antigen specific cytotoxic activity.

In one aspect of the invention said effector cell comprises a CD4 and/or CD8+ T cell or a precursor thereof. The specificity of HLA-restricted TCR recognition is defined by the α- and β chains of the TCR complex. Since the specific combination of the α- and β chain defines the specificity of a T cell, this specificity can be transferred from the parental cell to other effector cells by gene transfer of both genes coding for the α- and β chains. By RT-PCR, using primers that cover the complete repertoire of known TCR genes, the TCRα and β mRNA of specific T cell clones can be determined and cloned into retroviral vectors. As is exemplified in the experimental part of the present invention, we have isolated and cloned various α and β cDNA from major- and minor Histocompatibility antigen specific T cells. Such T cell clones as well as peptide specific T cell clones can be generated in unrelated selected individuals allowing selecting of TCR with optimal affinity and avidity. Unselected T (precursor) cells can be stimulated and transduced with retroviral supernatants coding for the specific TCRα or β chains by co-localization of the target cells and retrovirus on fibronectin resulting in a high transduction efficiency. By specific isolation of T cells containing both the TCRα and β chains based on the co-expression of marker genes, we demonstrate that unselected CD8 and CD4+ T cells could be transformed into antigen specific killer cells recognizing the target structure of interest. It was thus shown that both CD4 and CD8+ T cells that were initially *not* isolated on their bases of cytotoxicity could be transformed into cytolytic effector cells by activation and specific transfer of the TCRα and β complex and that these effector cells are capable of recognizing naturally processed antigen resulting in death of the target cell. Thus in embodiment of the invention said effector cell or a precursor thereof is an unselected CD8 and/or CD4[+] T cell. With unselected is meant that said cell has not undergone an antigen specific *in vitro* expansion and/or activation procedure prior to using said cell for a method of the invention. Unselected cells can be obtained directly or indirectly following culture, from the body of an individual. Said individual may have been subject to blood cell transplantation prior to said collection of unselected cells. Selection is defined as a deliberate act to obtain effector cells (that are of course antigen specific) through a method in vitro.

A method of the invention may further comprise providing said effector cell with another molecule. In one embodiment said effector cell is further provided with a CD4 molecule or a functional equivalent thereof. In another embodiment said effector cell is further provided with a CD8 molecule or a functional equivalent thereof. An effector cell lacking CD4 and CD8 may thus be provided with CD4 and/or CD8 functionality. A cell comprising CD4 or CD8 may thus be provided with an enhanced CD4 and/or CD8 functionality. In a preferred embodiment of the invention, a γδ T cell or a precursor thereof, is provided with a CD4 and/or CD8 molecule. Although at least some γδ-T cells expressed CD4 and/or CD8, antigen specific αβ T cell receptor mediated effector activity of a γδ-T cell obtained with a method of the invention, can be enhanced upon further providing said cell with a CD4 and/or CD8 molecule. Moreover, considering that at least some γδ-T cells do not express CD4 and/or CD8. It is possible, by providing said cell with either a CD4 or a CD8 molecule to generate an antigen specific γδ-T cell with different properties. CD4 expression enhances the recognition of HLA-class II molecules, whereas CD8 molecules promote the interaction with HLA-class I molecules.

A method of the invention may further comprise providing said (candidate) effector cell with a nucleic acid sequence encoding a safeguard molecule. A safeguard molecule is a molecule capable of conditionally inactivating an effector cell of the invention. Preferably said inactivating comprises killing of said effector cell. In a preferred embodiment safeguard molecule comprises a Herpes Simplex Virus thymidine kinase protein or a functional equivalent thereof. A safeguard molecule provides another level of safety for an effector cell of the invention. By providing the condition said safeguard molecule is capable of inactivating said effector cell. It is possible to at least in part neutralize an property of said effector cell. By providing a suitable substrate, such as gancyclovir, to a cell comprising a Herpes Simplex Virus thymidine kinase protein, it is possible to preferentially kill said cell. Conditional inactivation is preferably achieved in the body of an individual.

In another aspect the invention provides a cell obtainable by a method according to the invention. Preferably said cell is a T cell or a precursor thereof. In another aspect the invention provides a T cell or a precursor thereof provided with an $\alpha\beta$ T cell receptor or a functional part, derivative and/or analogue thereof. In a preferred embodiment said T cell comprises a $\gamma\delta$-T cell or a precursor thereof.

The hemopoietic system is a complex cell system, wherein primitive, undifferentiated stem cells, with extensive self-renewal capacity comprise the potential to differentiate into all of the various differentiated hemopoietic cells. This differentiation occurs through a cascade of more committed progenitor cells which have a more limited capacity of self-renewal and a more limited spectrum of hemopoietic cell types in which they can differentiate. Thus a precursor of a candidate effector cell of the invention can be any cell capable of differentiating into a candidate effector cell. Preferably, said precursor cell is a stem cell or a progenitor cell.

Individuals suffering from a human immuno-deficiency virus (HIV) infection typically are capable of mounting an effector response against HIV infected cells, at least during the early stages of infection. However, very often this effector response is not sufficient to effectively eradicate all of the infected cells from the body. One of the problems with effectively combating an HIV infection is that the virus naturally infects CD4+ T cells in the body. These cells are crucial to initiate and maintain an effector response. In the course of an infection the CD4 content of the blood of an individual drops until the individual is not capable of eliciting an effective effector response any more. The present invention now provides a method to generate an effector cell that is at least in part resistant to infection by HIV. This is achieved by providing a candidate effector cell, or precursor thereof, said cell lacking a co-receptor for HIV, with an $\alpha\beta$-T cell receptor. Preferably, said effector cell comprises a $\gamma\delta$-T cell or a precursor thereof. A cell of this embodiment of the invention is particularly suited for the treatment of an HIV-infected individual. Such cells can be used to combat HIV infected cells in said individual. However, said cells can also be used to combat other infections in HIV infected individuals. In another aspect the invention provides an effector cell generated from a CD4 negative $\gamma\delta$ cell. Such a cell lack the main receptor for HIV an can still display at least partial effector cell activity thus can be used to combat disease in an HIV infected patient.

In one aspect of the invention a cell of the invention is used for the preparation of a medicament. Preferably, said medicament is used in adoptive immuno therapy. More preferably, said medicament is used for the treatment of cancer and/or infectious disease.

In yet another aspect the invention provides a method for influencing immunity in an individual comprising administering to said individual a cell according to the invention.

With a method of the invention it is possible to generate a cell with a predefined antigen specificity. An $\alpha\beta$ T cell receptor can be very selective for an antigen and many different $\alpha\beta$ T cell receptors comprising many different specificities can easily be selected and cloned. One way to do this is for instance by cloning $\alpha\beta$ T cell receptors from a T cell with a known antigen specificities. Alternatively, libraries of T cell receptors or parts thereof can be screened for receptors comprising an appropriate antigen specificity. Thus effector cells can be generated comprising any antigen specificity.

A cell can be provided with an $\alpha\beta$ T cell receptor in various ways. For instance, an $\alpha\beta$ T cell receptor protein may be provided to said cell. This can be done using a T cell receptor comprising a hydrophobic moiety allowing insertion of the hydrophobic part in a membrane of said cell. Preferably said cell is provided with said T cell receptor through providing said cell with nucleic acid sequence encoding said T cell receptor. In this way the cell is provided with a continuous supply of new T cell receptor protein. Preferably, said nucleic acid sequence is integrated in the genome of said cell thus allowing continued presence of said T cell receptor after several cell divisions.

An $\alpha\beta$ T cell receptor of the invention, as mentioned above, comprises a specificity for an antigen. Preferably said T cell receptor comprises a specificity for an antigen presented in the context of an HLA molecule. Since there are many different HLA molecules, said $\alpha\beta$ T cell receptor can be specific for a particular antigen/HLA combination. This property can be used to generate an effector cell that is capable of discriminating between antigen expressing cells comprising different HLA molecules, such as for instance antigen expressing cells obtained from HLA mismatched individuals. Such allogeneic T cell receptors are very often more potent than autologous T cell receptors.

An antigen can be any molecule provided that a T cell receptor is capable of binding to it. Preferably, said antigen is expressed by a cell. Preferably, said antigen is specifically expressed in or on a particular cell type. In this way an effector cell of the invention is provided with a target cell specificity for said particular cell type. Effector function of a cell of the invention can thus be targeted toward this particular cell type. This property can be used for instance to induce tolerance toward a particular cell type in for instance auto-immune disease or transplantation. In another embodiment of the invention this property is used to direct cytotoxic activity toward this particular cell type. This can be used in transplantation, for instance to at least in part remove said particular cell type from a host receiving a transplant of this cell type from a donor not comprising said antigen and/or antigen/HLA complex. In a preferred embodiment effector activity of a cell of the invention is targeted toward a deleterious cell, such as a neoplastic (cancer) cell and/or a cell comprising a (part of) a microbial cell, virus or phage. Such targeting can initiate or amplify an immunity related process of removal of deleterious cells. In yet another embodiment an effector cell of the invention is provided to an individual suffering from an auto-immune disease.

An $\alpha\beta$ T cell receptor comprises a variable part capable of recognizing antigen in the context of an MHC molecule and a constant part that serves to anchor the $\alpha\beta$ T cell receptor in a membrane of the cell and that further serves to transmit a signal in response to a binding event in the variable part to a CD3 molecule. For the present invention a functional part of an $\alpha\beta$ T cell receptor comprises at least an antigen binding part of said variable domain. Said functional part of course needs to be anchored in a cellular membrane. This can be achieved in a variety of ways. A non-limiting example is a fusion of said functional part with a membrane bound part of a protein. These chimeric receptors composed of the variable regions of the TCR$\alpha$ and $\beta$ chains can be linked to several different membrane bound molecules, including the FcRI$\gamma$ chain, CD4, CD8 and the CD3 $\varsigma$ chain. Another option would be to repalce the variable regions of the $\gamma$ and $\delta$ chain of the TCR $\gamma\delta$ complex with the variable regions of the TCR $\alpha$ and $\gamma$ chains.

An $\alpha\beta$ T cell receptor or a functional part thereof may be provided by providing a separate $\alpha$ and $\beta$ chain (or parts thereof). Alternatively, said $\alpha\beta$ T cell receptor is provided as a single chain, for instance through a so-called single chain $\alpha\beta$ T cell receptor (Weijtens, 1998). In a preferred embodiment, a functional part of an $\alpha\beta$ T cell receptor further comprises a part of said receptor capable of interacting with a CD3 molecule. In this way a functional $\alpha\beta$ T cell receptor signaling pathway can be provided to a cell comprising said CD3 molecule.

Derivatives of molecules identified in the present application are molecules comprising the same function in kind not necessarily in amount. Such molecules can for instance be obtained through conservative amino-acid substitution. Other methods of arriving at different, though similar molecules comprising the same activity in kind are known to a person skilled in the art and are for the present invention considered derivatives. An analogue of a molecule of the present invention comprises the same function in kind as the molecule it is an analogue of. An analogue does not necessarily need to comprise the same amount of activity. Analogues molecules are for instance homologues molecules derived from one species that are used in (a cell of) another species.

Examples

*Materials and Methods:*

*Retroviral vectors:*

By RT-PCR, using primers that cover the total repertoire of known TCR chains, the TCR $\alpha$ and $\beta$ usage of the mHag or major specific T cell clones were determined, and cloned into retroviral vectors. The Moloney murine leukemia virus based retroviral vector LZRS and packaging cells $\varphi$-NX-A were used for this purpose (kindly provided by G. Nolan). The LZRS vector contains the puromycin resistance gene, which facilitates the selection of transfected producer cells and the EBNA-1 sequence, which maintains the retroviral vectors as episomes within the packaging cell line. This affords a reproducible rapid, large-scale, and high-titer retroviral production. Two bicistronic retroviral vectors were constructed in which the multiple cloning site is linked to the downstream internal ribosome entry sequence (IRES) and the marker gene green fluorescent protein (GFP) or truncated form of the nerve growth factor ($\Delta$NGF-R). These two retroviral vectors make it feasible to perform co-transductions with two retroviral vectors coding for two genes of interest in combination with different marker genes. Retroviral vectors were constructed encoding the TCR $\alpha$ chains of different antigen specific T cell clones in combination with GFP and the TCR $\beta$ chains in combination with the $\Delta$NGF-R.

*Retroviral transduction of $\alpha\beta$ and $\gamma\delta$ T cells*

Unselected $\alpha\beta$ positive T cells derived from peripheral blood were stimulated with PHA (800 ng/ml) and IL-2 (120 IU/ml) at a concentration of $0.5 \times 10^6$ cells/ml. After two days of culture the T cells were transduced with retroviral supernatant. The $\gamma\delta$ T cells were isolated from peripheral blood by depletion of all $\alpha\beta$ positive T cells using autoMACS separation, and the $\gamma\delta$ T cells were cultured in the presence of IL-2 (300 IU/ml) at a concentration of $0.5 \times 10^6$ T cells/ ml and transduced with retroviral supernatant after 2 days of culture. The transduction procedure used for these two T cell types was based on the method developed by Hanenberg *et al.* using recombinant human fibronectin fragments CH-296. Non-tissue culture treated Falcon petridishes (3 cm) (Becton Dickinson) were coated with 1 ml of 30 $\mu$g/ml recombinant human fibronectin fragment CH-296 (Takara Shuzo Co., Japan) at room temperature (RT) for 2 h. The CH-296 solution was removed and petridishes were blocked with 2 % of human serum albumin for 30 min at RT. Petridishes were washed and T cells were added (max. $5 \times 10^6$ cells/ petridish) together with 1 ml of thawed retroviral supernatant. Cells were cultured at 37°C for 6 h or overnight, washed and transferred to 24-well culture plates.

*Cytotoxicity assay.*

Target cells were harvested and labeled with 50 µCi $Na_2{}^{51}CrO_4$ for 60 min at 37°C. Target cells were added to various numbers of effector cells, in a final volume of 150 µl IMDM supplemented with fetal calf serum in 96-well U-bottomed microtiter plates. After 6 h or overnight incubation at 37°C, 25 µl of supernatant was harvested and measured in a scintillation counter. The mean percentage of specific lysis of triplicate wells was calculated as follows:

specific lysis = [(experimental release - spontaneous release)/(maximal release

- spontaneous release)] x 100.

*Flow cytometric analysis and sorting:*

The transduction efficiency, measured by the expression of the markers GFP and NGF-R, was analyzed by FACS, 3 to 4 days after transduction. In addition, FACS analyses were performed using specific mAbs to test for the expression of the specific TCR α and β chains and for the CD8α (BD) on γδ T cells. $10^5$ cells were washed with ice-cold PBS with 0.1% BSA and 0.01% azide. Cells were incubated for 30 min at 4°C with the specific mAbs, washed and if necesarry labeled with goat anti mouse PE. Cells were washed and analyzed on a FACScan (BD). Transduced T cells were FACS sorted on basis of GFP+Δ NGF-R+, for the transduced γδ T cells the sorting on GFP+ ΔNGF-R+ was combined with sorting on TCR γδ positive T cells.

*Results:*

*Retroviral gene transfer of TCR α and β chains into αβ T cells*

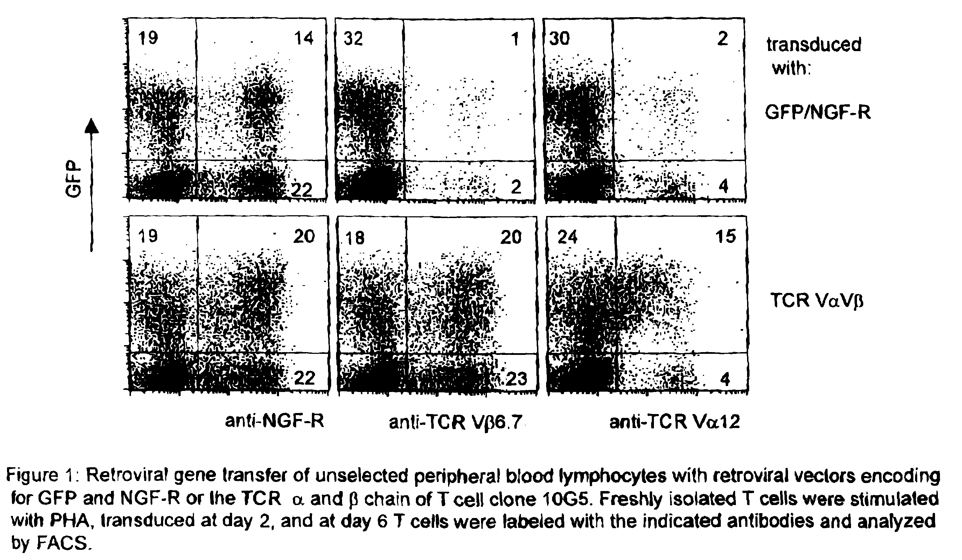

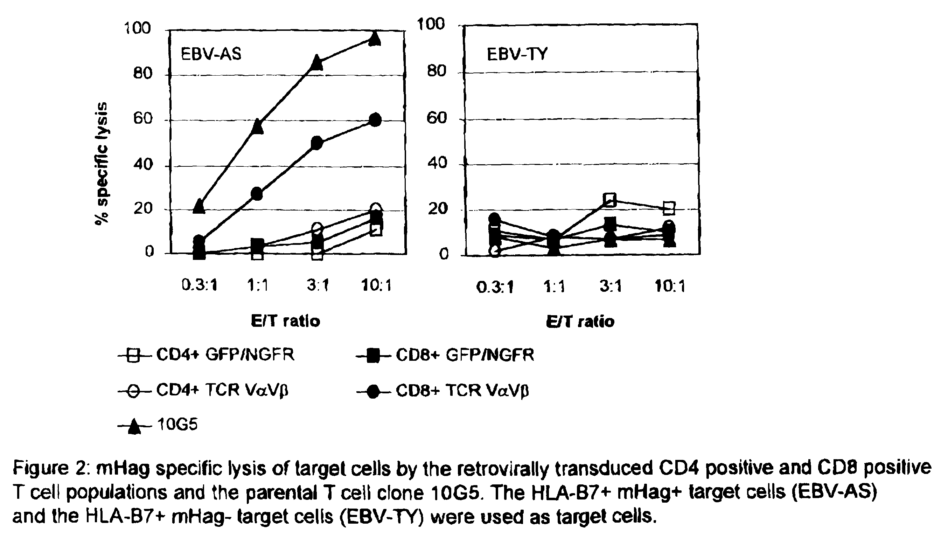

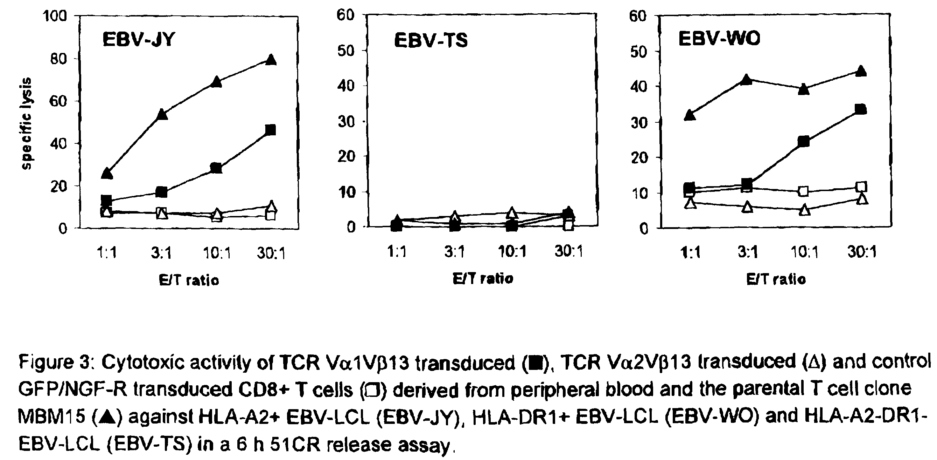

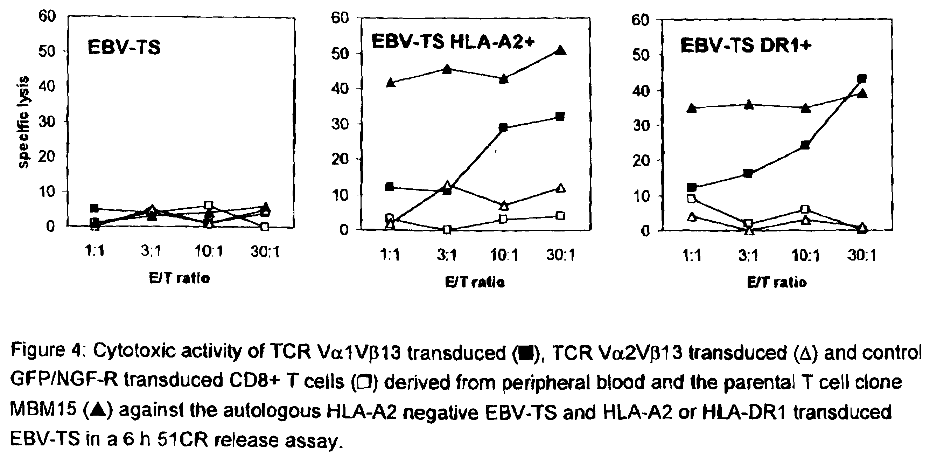

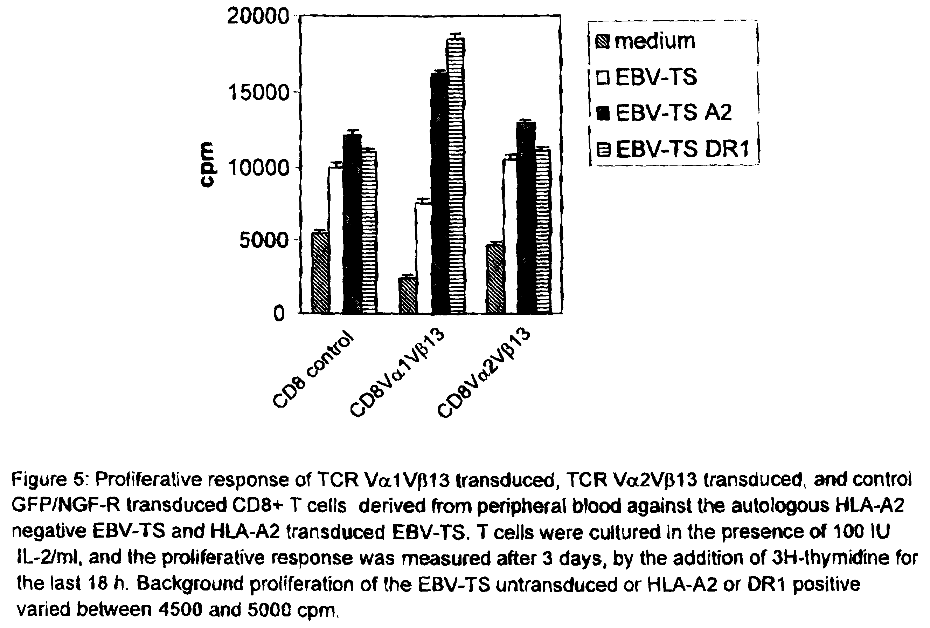

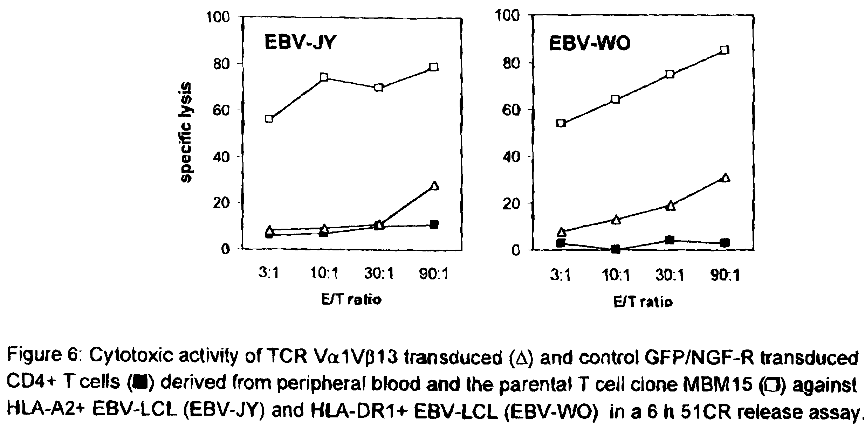

To rescue TCR specificity of antigen specific T cells and to explore their functional characterization, TCR α and β genes, derived from major or minor histocompatibility antigen (mHag) specific T cell clones, were transferred into T cell populations with a high proliferative capacity. This method gives the opportunity to preserve the antigen specificities of T cell clones and characterize the fine specificity of the TCR. First, we transduced PHA stimulated T cells derived from freshly isolated peripheral blood with the TCR α and β genes derived from the mHag specific HLA class I restricted T cell clone 10G5. This T cell clone, generated in an HLA identical setting, was characterized as being an HLA-B7 restricted, mHag specific T cell clone. The molecular nature of the mHag that is recognized by this T cell clone has not yet been identified. Two retroviral vectors were constructed, one encoding the 10G5 derived TCR Vα12 gene in combination with GFP and the other vector encoding the 10G5 derived TCR Vβ6.7 gene in combination with the truncated form of the nerve growth factor receptor (ΔNGF-R) as marker gene. Double transduction of PHA stimulated unselected T cells derived from freshly isolated unrelated peripheral blood with these retroviral supernatants demonstrated that 50-60% of the total T cell population was retrovirally transduced. Of these retrovirally transduced T cells, 15-20% coexpressed GFP and ΔNGF-R (figure 1), indicating that these T cells were transduced with both retroviral vectors. The GFP and ΔNGF-R expression was stable and appeared in both the CD4+ and CD8+ T cell populations. Antibody staining for the specific TCRα chain (TCR Vα12.1) showed that it was expressed at the cell surface of GFP positive T cells (figure 1). In addition, 40% of the T cells was positive for the TCRβ chain (TCR Vβ6.7) specific antibody, correlating well with the percentage of ΔNGF-R positive T cells.
The GFP/ΔNGF-R expressing CD4 positive T cells and GFP/ΔNGF-R expressing CD8 positive T cells were sorted by FACS, expanded and tested in a 51Cr release assay. EBV transformed B cells (EBV-LCL) expressing the HLA class I restriction element HLA-B7 and the mHag (EBV-AS) and EBV-LCL expressing HLA-B7 but not the mHag (EBV-TY) were used as target cells. As positive control the parental T cell clone 10G5 was included in these experiments (figure 2). The results demonstrated that the TCR VαVβ transduced CD8 positive T cell population specifically recognized the HLA-B7 expressing, mHag positive target cells almost as effective as the parental T cell clone 10G5. In contrast, no specific lysis was observed with the GFP/ΔNGF-R control transduced CD8 positive T cells. Both the TCR VαVβ and GFP/ ΔNGF-R transduced CD4+ T cells were not able to specifically lyse the HLA-B7+ mHag+ target cells. These results combined with the surface expression of the TCR Vα and Vβ demonstrated that we have efficiently transferred a functional HLA-class I restricted TCRαβ complex to human CD8+ T cells, in order to redirect their specificity. Furthermore, the retrovirally transduced T cells (both GFP/ΔNGF-R control as TCR VαVβ transduced T cells) expanded after aspecific stimulation vigorously (doubling time = 1 day) *in vitro,* indicating that these transduced T cells are ideal tools to use for characterization of the specificity of the TCR or for future clinical use. Furthermore, we have shown that the functionality of the transferred TCR is stable thoughout a culture period of at least 21 days.
In addition, we transduced PHA stimulated **T** cells derived from freshly isolated peripheral blood with the TCR α and β genes derived from the major specific HLA class I restricted T cell clone MBM15. This T cell clone, generated in an haploidentical mixed lymphocyte reaction, was characterized as being an HLA-A2 restricted, major specific T cell clone. Interestingly, besides the HLA-A2 restricted alloreactivity, this T cell clone exerts also an HLA-DR1 restricted recognition. Based on the fact that we identified by RT-PCR two in-frame TCR Vα gene transcripts and one TCR Vβ gene transcript, three retroviral vectors were constructed, encoding the MBM15 derived TCR Vα1 or Vα2 gene in combination with GFP and a retroviral vector encoding the MBM15 derived TCR Vβ13 gene in combination with ΔNGF-R. PHA stimulated unselected T cells derived from freshly isolated unrelated peripheral blood and negative for HLA-A2 and DR1, were transduced with the combinations of TCR Vα1 and Vβ 13 or TCR Vα2 and Vβ13. Antibody staining for TCR Vβ13 demonstrated that it was expressed at the cell surface of T cells transduced with both TCR combinations on the ΔNGF-R positive T cells (data not shown). No specific mAbs were available for both the TCR Vα chains. Double positive GFP/ΔNGF-R cells of both transductions and control GFP/ΔNGF-R transduction were sorted by FACS and tested for functionality in cytotoxicity assays, to investigate whether we were able to reproduce the retroviral transfer of the TCR genes derived from the mHag specific T cell clone 10G5. Furthermore, we were interested whether both TCR complexes were essential for the dual recognition or one of the two TCR complexes. HLA-A2+ EBV-LCL, HLA-DR1+ EBV-LCL and control HLA-A2 and HLA-DR1 negative EBV-LCL were used as target cells in 51Cr release assays. As positive control the parental T cell clone MBM15 was included in these experiments (figure 3). The results demonstrated that the TCR Vα1Vβ13 transduced CD8+ T cell population specifically recognized the HLA-A2 or HLA-DR1 expressing target cells almost as effective as the parental T cell clone MBM15. In contrast, no specific lysis was observed with the TCR Vα2Vβ13 and GFP/ΔNGF-R control transduced CD8 positive T cells. In addition, autologous EBV-LCL negative for HLA-A2 and HLA-DR1, and therefore not recognized by the parental T cell clone MBM15, were transduced with HLA-A2 or HLA-DR1 and used as target cells. The results shown in figure 4 indicate that both MBM15 as well as TCR Vα1V β13 transduced CD8+ T cells lysed the HLA-A2 and HLA-DR1 transduced EBV-TS, demonstrating that the dual recognition of MBM15 was mediated via the TCR Vα1Vβ13 complex and efficiently transferred into non-selected CD8+ T cells. In addition, we also observed specific proliferation of the TCR Vα1Vβ13 transduced CD8+ T cells after stimulation with the HLA-A2 and HLA-DR1 transduced autologous EBV-LCL, in comparison with TCR Vα2Vβ13 and control transduced CD8+ T cells (figure 5), Furthermore, CD4+ T cells transduced with the TCR Vα1Vβ13 were able to mediate specific lysis of both the HLA-A2 positive and HLA-DR1 positive target cells, although the lytic capacity was lower compared to the lytic capacity of the TCR Vα1Vβ13 expressing CD8+ T cells (figure 6). After overnight incubation the lytic activity of the TCR Vα1Vβ13 transduced CD4+ T cells was more pronounced (data not shown). FACS analysis excluded contamination of CD8+ T cells in these retrovirally transduced CD4+ T cell populations. Importantly, the functionality of the transferred TCR, measured by specific cytotoxicity and proliferative capacity was stable for more than 1.5 month in both the CD4+ and CD8+ T cell populations. These data demonstrate that we are able to efficiently redirect the specificity of different T cell populations by introducing TCR genes derived from antigen specific T cell clones.

*Retroviral gene transfer of TCR α and β chains into γδ T cells*

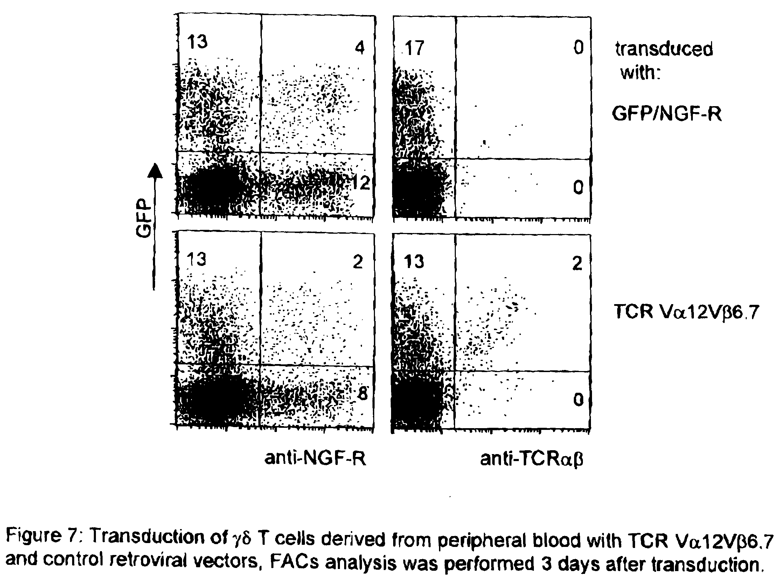

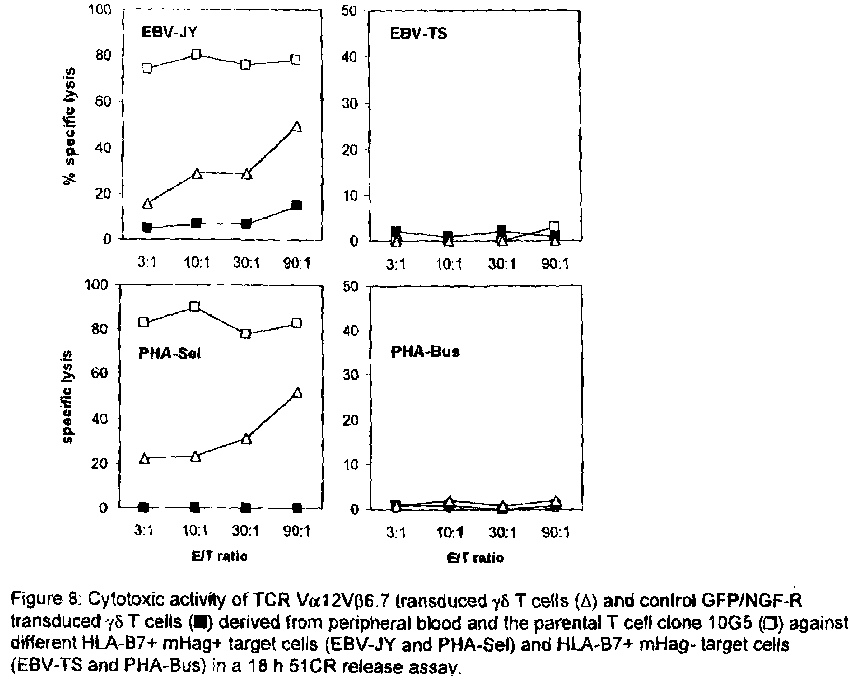

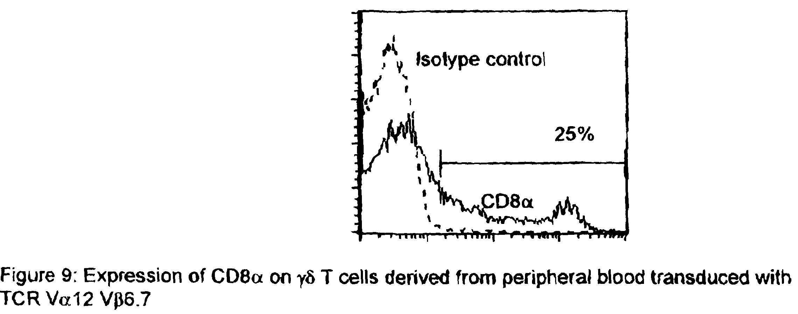

To rescue TCR specificity of mHag or leukemia reactive T cells and to redirect the TCR specificity of different cell populations, the TCR α and β genes derived from mHag and leukemia specific T cells, can be cloned and transferred into several different cell populations. The most obvious candidate for TCR directed gene transfer is the αβ T cell derived from peripheral blood as described above. These T cells have the complete intracellular machinery to express a functional TCRαβ complex at the cell surface, the cells express CD3 and the appropriate co-receptors CD4 or CD8. In addition, these cells have a normal expression pattern of costimulatory and adhesion molecules needed for recognition of target cells, and a high proliferative capacity. However, because of the endogenous expression of TCR α and β chains, new TCRαβ complexes with unknown specificity will arise due to pairing of the retrovirally and endogenous expressed TCR α and β chains. To circumvent this problem, γδ T cells derived from peripheral blood may be alternative candidates. γδ T cells express CD3 to transport the TCRαβ complex to the cell surface, and do not have rearranged TCR α and β chains. In addition, part of the γδ T cells also express the CD4 or CD8 molecule. To investigate this possibility, the TCR α and β genes derived from the above described mHag specific HLA-B7 restricted 10G5, were introduced into γδ T cells derived from peripheral blood of an unrelated healthy donor. The γδ T cells were stimulated with IL-2 and retrovirally transduced after two days of culture with the retroviral vectors coding for the TCR α and β genes derived from the 10G5 T cell clone. FACS analysis performed after 3 days of transduction demonstrated that we were able to efficiently transduce these peripheral blood derived γδ T cells with the retroviral vectors varying from 20-25% (figure 7). Interestingly, cell surface expression of TCRαβ complexes on γδ T cells was only observed when the T cells were double positive for the marker genes GFP and ΔNGF-R. This result indicate that both TCR α and β chain are needed for transport to the cell surface to form a TCRαβ complex. By FACS sorting the GFP/ΔNGF-R positive γ δ T cells were isolated and expanded by aspecific stimulation using irradiated allogeneic PBMCs, PHA and IL-2. The γδ T cells transduced with the TCR genes derived from the mHag specific T cell clone 10G5 were tested against EBV-LCL and PHA blasts expressing the HLA class I restriction element HLA-B7 and the mHag and EBV-LCL and PHA blasts expressing HLA-B7 but not the mHag. As positive control the parental T cell clone 10G5 was included in these experiments (figure 8). The results demonstrated that the TCR V$\alpha$V$\beta$ transduced $\gamma\delta$ T cell population specifically recognized the HLA-B7 expressing, mHag positive target cells. In contrast, no specific lysis was observed with the GFP/$\Delta$NGF-R control transduced $\gamma\delta$ T cells. Antibody staining for CD8$\alpha$ indicated that 25% of the retrovirally transduced $\gamma\delta$ T cells expressed the CD8$\alpha$ molecule at the cell surface (figure 9). In addition, similar as for the $\alpha\beta$ positiveT cells mentioned above, the retrovirally transduced $\gamma\delta$ T cells (both GFP/$\Delta$NGF-R control as TCR V$\alpha$V$\beta$ transduced T cells) expanded vigourously after aspecific stimulation (doubling time = 2 day) *in vitro,* indicating that these transduced T cells are ideal tools for future clinical use. These results together demonstrate that we are able to generate $\gamma\delta$ T cells with high proliferative capacity and with HLA restricted antigen specific killing capacity of the transferred TCR $\alpha\beta$ complex.

References

Weijtens, IMEN; Willemsen, R.A.; Hart, E.H. and Bolhuis, RLH. A retroviral vector system "STITCH" in combination with an optimized single chain antibody chimeric receptor gene structure allows efficient gene transduction and expression in human T lymphocytes: Gene Therapy (1998) 5: 1195-1203.

**Claims**

1. A method for generating an effector cell comprising providing a candidate effector cell or a precursor thereof, with a recombinant $\alpha\beta$ T cell receptor or a functional part, derivative and/or analogue of said receptor.

2. A method according to claim 1, wherein said candidate effector cell is a primary cell.

3. A method according to claim 1 or claim 2 wherein said cell is obtainable from bone marrow, a lymphoid organ or blood.

4. A method according to any one of claim 1-3, wherein said cell is a $\gamma\delta$-cell.

5. A method according to any one of claims 1-4, wherein said effector cell is a cytotoxic T cell and/or cytokine-producing cell.

6. A method according to any one of claims 1-5, wherein said candidate effector cell does not express a functional endogenous T cell receptor $\alpha$ and/or $\beta$ chain.

7. A method according to any one of claim 1-6, wherein said recombinant $\alpha\beta$ T cell receptor is derived from a cell.

8. A method according to claim 7, wherein said recombinant $\alpha\beta$ T cell receptor comprises a capability of binding to an antigen presented in the context of an allogeneic HLA molecule.

9. A method according to any one of claims 1-8, further comprising providing said cell with a CD4 molecule or a functional equivalent thereof.

10. A method according to any one of claims 1-9, further comprising providing said cell with a CD8 molecule or a functional equivalent thereof.

11. A method according to anyone of claims 1-10, further comprising providing said cell a nucleic acid sequence encoding a safeguard molecule.

12. A method according to claim 11, wherein said safeguard molecule comprises a Herpes Simplex Virus thymidine kinase protein or a functional equivalent thereof

13. A cell obtainable by a method according to any one of claims 1-12.

14. A candidate effector cell or a precursor thereof, provided with an $\alpha\beta$ T cell receptor or a functional part, derivative and/or analogue thereof.

15. A γδ T cell or a precursor thereof, comprising an αβ T cell receptor or a functional part, derivative and/or analogue thereof.

16. A cell according to any one of claims 13-15, lacking a (co-)receptor for human immuno deficiency virus.

17. Use of a cell according to any one of claims 13-16, for the preparation of a medicament.

18. Use of a cell according to anyone of claims 13-16, for the preparation of a medicament for the treatment of cancer and/or infectious disease.

19. A method for influencing immunity in an individual comprising administering to said individual a cell according to anyone of claims 13-16.

Figure 1: Retroviral gene transfer of unselected peripheral blood lymphocytes with retroviral vectors encoding for GFP and NGF-R or the TCR α and β chain of T cell clone 10G5. Freshly isolated T cells were stimulated with PHA, transduced at day 2, and at day 6 T cells were labeled with the indicated antibodies and analyzed by FACS.

Figure 2: mHag specific lysis of target cells by the retrovirally transduced CD4 positive and CD8 positive T cell populations and the parental T cell clone 10G5. The HLA-B7+ mHag+ target cells (EBV-AS) and the HLA-B7+ mHag- target cells (EBV-TY) were used as target cells.

Figure 3: Cytotoxic activity of TCR Vα1Vβ13 transduced (■), TCR Vα2Vβ13 transduced (Δ) and control GFP/NGF-R transduced CD8+ T cells (□) derived from peripheral blood and the parental T cell clone MBM15 (▲) against HLA-A2+ EBV-LCL (EBV-JY), HLA-DR1+ EBV-LCL (EBV-WO) and HLA-A2-DR1- EBV-LCL (EBV-TS) in a 6 h 51CR release assay.

Figure 4: Cytotoxic activity of TCR Vα1Vβ13 transduced (■), TCR Vα2Vβ13 transduced (Δ) and control GFP/NGF-R transduced CD8+ T cells (□) derived from peripheral blood and the parental T cell clone MBM15 (▲) against the autologous HLA-A2 negative EBV-TS and HLA-A2 or HLA-DR1 transduced EBV-TS in a 6 h 51CR release assay.

EP 1 188 825 A1

Figure 5: Proliferative response of TCR Vα1Vβ13 transduced, TCR Vα2Vβ13 transduced, and control GFP/NGF-R transduced CD8+ T cells derived from peripheral blood against the autologous HLA-A2 negative EBV-TS and HLA-A2 transduced EBV-TS. T cells were cultured in the presence of 100 IU IL-2/ml, and the proliferative response was measured after 3 days, by the addition of 3H-thymidine for the last 18 h. Background proliferation of the EBV-TS untransduced or HLA-A2 or DR1 positive varied between 4500 and 5000 cpm.

EP 1 188 825 A1

Figure 6: Cytotoxic activity of TCR Vα1Vβ13 transduced (△) and control GFP/NGF-R transduced CD4+ T cells (■) derived from peripheral blood and the parental T cell clone MBM15 (□) against HLA-A2+ EBV-LCL (EBV-JY) and HLA-DR1+ EBV-LCL (EBV-WO) in a 6 h 51CR release assay.

EP 1 188 825 A1

Figure 7: Transduction of γδ T cells derived from peripheral blood with TCR Vα12Vβ6.7 and control retroviral vectors, FACs analysis was performed 3 days after transduction.

EP 1 188 825 A1

Figure 8: Cytotoxic activity of TCR Vα12Vβ6.7 transduced γδ T cells (Δ) and control GFP/NGF-R transduced γδ T cells (■) derived from peripheral blood and the parental T cell clone 10G5 (□) against different HLA-B7+ mHag+ target cells (EBV-JY and PHA-Sel) and HLA-B7+ mHag- target cells (EBV-TS and PHA-Bus) in a 18 h 51CR release assay.

Figure 9: Expression of CD8α on γδ T cells derived from peripheral blood transduced with TCR Vα12 Vβ6.7

**European Patent Office**

## PARTIAL EUROPEAN SEARCH REPORT

which under Rule 45 of the European Patent Convention shall be considered, for the purposes of subsequent proceedings, as the European search report

Application Number

EP 00 20 3232

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X<br>Y | CA 2 205 881 A (INSERM)<br>9 January 1999 (1999-01-09)<br>* page 7, line 20 – page 8, line 5;<br>claims; examples 4,5 *<br>* page 5, line 1 – line 8 * | 1-10,<br>13-19<br>11,12 | C12N5/10<br>A61K35/14<br>A61P35/00<br>A61P31/00 |
| Y | B.K. KERALAVARMA ET AL.: "Improved "suicide" retroviral vector containing mutant HSV-tk gene confers increased sensitivity to gancyclovir."<br>BLOOD,<br>vol. 94, no. 10, Suppl 1 (Part 2 of 2),<br>15 November 1999 (1999-11-15), page 329b<br>XP002170636<br>New York, N.Y., US<br>* abstract # 4698 * | 11,12 | |
| A | WO 00 44893 A (PALMETTO RICHLAND MEMORIAL HOSPITAL) 3 August 2000 (2000-08-03)<br>* page 7, line 24 – page 8, line 13;<br>claims * | 1-19 | **TECHNICAL FIELDS SEARCHED (Int.Cl.7)**<br><br>C12N<br>C07K |

### INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

Although claim 19 is directed to a method of treatment of the human/animal body (Article 52(4) EPC), the search has been carried out and based on the alleged effects of the compound/composition.

| Place of search | Date of completion of the search | Examiner |
|---|---|---|
| THE HAGUE | 27 June 2001 | Ryckebosch, A |

EPO FORM 1503 03 82 (P04C07)

**ANNEX TO THE EUROPEAN SEARCH REPORT
ON EUROPEAN PATENT APPLICATION NO.** EP 00 20 3232

This annex lists the patent family members relating to the patent documents cited in the above-mentioned European search report.
The members are as contained in the European Patent Office EDP file on
The European Patent Office is in no way liable for these particulars which are merely given for the purpose of information.

27-06-2001

| Patent document cited in search report | | Publication date | Patent family member(s) | | Publication date |
|---|---|---|---|---|---|
| CA 2205881 | A | 09-01-1999 | NONE | | |
| WO 0044893 | A | 03-08-2000 | AU | 3472800 A | 18-08-2000 |